# EUROPEAN PATENT SPECIFICATION

(11) **EP 2 168 561 B1**
(45) Date of publication and mention of the grant of the patent: **23.11.2011**
(21) Application number: 09012413.2
(22) Date of filing: 30.09.2009
(51) Int. Cl.: A61K 6/027, A61K 6/04, A61K 6/083

(54) **Organic and inorganic composite filler**
Füllstoff Materialen mit organischen und anorganischen Stoffen
Composite comprenant des fillers inorganiques et organiques.

(30) Priority: 30.09.2008 JP 2008253904
(43) Date of publication of application: 31.03.2010
(73) Proprietor: GC Corporation, Itabashi-ku Tokyo 174-8585 (JP)
(72) Inventor: Fusejima, Futoshi, Tokyo 174-8585 (JP); Kaga, Shinji, Tokyo 174-8585 (JP)
(74) Representative: Müller-Boré & Partner Patentanwälte

(56) References cited:
- EP-A2- 1 905 414
- EP-A2- 1 974 713
- WO-A1-2006/050829

## Description

The present invention relates to an organic and inorganic composite filler which is proper as a filler used for a dental restoration material having X-ray imaging property.

A general dental restoration material composite includes a monomer as a base material at polymerization, a filler such as glass powder, and a polymerization catalyst for curing the monomer. As for the glass powder as the filler, glass powder having an average particle diameter from 0.1 to 5 µm has been used. The dental restoration material composite called a dental composite resin is required to have X-ray imaging property for confirmation after a treatment. The X-ray imaging property is given to a dental restoration material composite after curing by blending a material having the X-ray imaging property to the glass powder.

However, when the glass powder having an average particle diameter from 0.1 to 5 µm is used, there is a problem that the dental restoration material composite tends to be "sticky", that is, when the dental restoration material composite is filled into a cavity of a tooth using a special spatula, the dental restoration material composite sticks to the spatula, so that there is a problem in operativity. In addition, a lot of monomer component is required in the dental restoration material composite, because the glass powder has a large specific surface area of a particle. Thus, the dental restoration material composite has a low ratio of the glass powder therein and, as a result, there are problems that polymerization shrinkage of the dental restoration material composite after curing increases, and the dental restoration material composite after curing has low mechanical strength and low X-ray imaging property.

As for a means for improving the problem of "sticky" and the problem of polymerization shrinkage, for example, Japanese Patent Application Laid-Open No. 5-194135 discusses a dental restoration material composite using an organic and inorganic composite filler having an average particle diameter from 5 to 50µm, which is produced by mixing glass powder having a maximum particle diameter of 10µm or less and an average particle diameter from 0.1 to 5µm with a monomer of methacrylate or acrylate, polymerizing and curing the monomer, and pulverizing the cured material. However, in the dental restoration material composite using only the organic and inorganic composite filler produced by the glass powder as a filler, a content of inorganic materials in the filler becomes' remarkably low. Thus, a thermal expansion coefficient increases so as to cause marginal leakage and also decrease mechanical strength. Therefore, this dental restoration material composite should be used together with other filler such as a glass powder and colloidal silica. As a result, there is a problem that the face of the dental restoration material composite after curing is not smooth. In addition, since the organic and inorganic composite filler using the glass powder is easily influenced by refractive index, the refractive index of the glass powder needs to match with the refractive index of a monomer after polymerization. However, since the refractive index of the monomer of methacrylate or acrylate is changed by polymerizing and curing, the relationship between the refractive indexes of the glass powder and the monomer is changed before and after curing, and thus there is a problem that transparency decreases.

On the other hand, for example, Japanese Translation of PCT Publication No. 2003- 512407 introduces a dental material in which silica particles having a nano-size and a material such as heavy metal oxide particles, are directly blended into the composite as fillers in order to give X-ray imaging property. However, when the average diameter of the heavy metal oxide particle is less than 0.1µm, there is a problem that a dental restoration material composite after curing cannot have a transparency similar to that of a natural tooth because of the difference between the refractive indexes of the oxide particles and a cured monomer. In addition, when only an inorganic filler is directly blended into the composite without blending the organic and inorganic composite filler, there remains a problem that the composite has low operativity which is a fault of a conventional fine particle filler.

The present applicant previously solved aforementioned problems as shown in Japanese Patent Application Laid-Open No. 2008-81469, by developing an organic and inorganic composite filler having an average particle diameter from 5 to 50µm produced by mixing a fine particle filler having an average particle diameter from 0.005 to 0.3µm and X-ray impermeability with a (meth)acrylate monomer, curing the mixture, and pulverizing the cured mixture. However, when a dental restoration material composite using the organic and inorganic composite filler is used, there may be a problem that mechanical strength such as bending strength and X-ray imaging property is insufficient.

The present invention is directed to provide an organic and inorganic composite filler to yield excellent surface smoothness similar to that of a natural tooth and to cause low polymerization shrinkage when used for a dental restoration material composite, and to acquire a dental restoration material composite having high X-ray imaging property and high mechanical strength.

Present inventors carried out earnest works to solve the aforementioned problems and, as a result, they found out the followings to complete the present invention. An organic and inorganic composite filler is produced by mixing glass powder having a specific particle diameter and X-ray imaging property and a metal compound having a specific particle diameter and X-ray imaging property, mixing the mixture to a (meth)acrylate compound, curing the mixture, and pulverizing the cured mixture, where 50 to 80% by weight of the glass powder having X-ray imaging property and 10 to 40% by weight of the metal compound having X-ray imaging property are mixed so as to make a total of the both to be 90% by weight at the maximum and have high density. Thus, excellent surface smoothness is given by the glass powder in fine particle. Further, since it is not necessary to separately use a lot of filler in fine particle, polymerization shrinkage of a dental restoration material composite after curing is hardly caused. Further, since the glass powder and the metal compound are filled with a high density, the dental restoration material can have high mechanical strength. Since a lot of the glass powder in fine particle is used, an influence by refractive index is reduced. Furthermore, since a lot of a powder having X-ray imaging property is filled in the organic and inorganic composite filler, sufficient X-ray imaging property is given.

According to an aspect of the present invention, an organic and inorganic composite filler used for a dental restoration material is produced by curing and pulverizing a (meth) acrylate compound including (a) 50 to 80% by weight of glass powder having a maximum particle diameter of 5µm or less, an average particle diameter from 0.05 to 2µm, and X-ray imaging property, and including (b) 10 to 40% by weight of a metal compound having an average particle diameter from 0.005 to 0.3µm and X-ray imaging property, where the (meth)acrylate compound includes 90% by weight of a combination of the glass powder (a) and the metal compound (b) at the maximum. The organic and inorganic composite filler has preferably an average particle diameter from 10 to 30µm.

The organic and inorganic composite filler for a dental restoration material according to the present invention is produced by curing and pulverizing a (meth)acrylate compound including 50 to 80% by weight of glass powder having a maximum particle diameter of 5µm or less, an average particle diameter from 0.05 to 2µm, and X-ray imaging property, and including 10 to 40% by weight of a metal compound having an average particle diameter from 0.005 to 0.3µm and X-ray imaging property, where the (meth)acrylate compound includes 90% by weight of both the glass powder and the metal compound at the maximum. Therefore, an influence by refractive index is reduced. When the organic and inorganic composite filler is used for a dental restoration material, a dental restoration material composite having excellent surface smoothness similar to that of a natural tooth, low polymerization shrinkage, high mechanical strength, and sufficient X-ray imaging property can be acquired.

A (meth) acrylate compound used in the organic and inorganic composite filler according to the present invention is a monomer or comonomer of methacrylate or acrylate and its oligomer or prepolymer. More particularly, methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-methoxyethyl (meth)acrylate, 2-ethylhexyl (meth)acrylate, benzyl (meth)acrylate, 2-hydroxy-1,3-di(meth)acryloxypropane, ethyleneglycol di(meth)acrylate, diethyleneglycol di(meth)acrylate, triethyleneglycol di(meth)acrylate, butyleneglycol di(meth)acrylate, neopentylglycol di(meth)acrylate, 1,3-butanediol di(meth)acrylate, 1,4-butanediol di(meth)acrylate, 1,6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutyleneglycol di(meth)acrylate, bisphenol A diglycidyl (meth)acrylate, or its comonomer, can be used. Further, as for (meth) acrylate having an urethane bond, di-2-(meth)acryloxyethyl 2,2,4-trimethylhexamethylenedicarbamate, and 1, 3, 5-tris[1,3-bis{(meth) acryloyloxy}-2-propoxycarbonyl aminohexane]-1,3,5-(1H, 3H, 5H) triazine-2,4,6-trion, can be used. In addition, (meth) acrylate of a urethane oligomer including 2,2'-di(4-hydroxycyclohexyl) propane, 2-oxypanone, hexamethylenediisocyanate, and 2-hydroxyethyl (meth)acrylate, and (meth)acrylate of a urethane oligomer including 1,3-butanediol, hexamethylenediisocyanate, and 2-hydroxyethyl (meth)acrylate, can be used.

These methacrylate and acrylate compounds are publicly known as a dental material, so that these can be used independently or by mixing depending on necessity.

As for a composition of glass powder having a maximum particle diameter of 5µm or less, an average particle diameter from 0.05 to 2µm, and X-ray imaging property, a glass including an alkaline earth metal atom such as calcium, strontium, or barium, which has X-ray imaging property, a zinc glass, and a lead glass, can be used. Particularly, a fluoroaminosilicate glass powder including aluminum oxide, anhydrous silicic acid, calcium fluoride, calcium phosphate, and strontium carbonate as raw materials can be preferably used because of having fluorine ion sustained-release property. The refractive index of a glass having X-ray imaging property is preferably within a range from 1.48 to 1.54, although depending on a kind of a (meth) acrylate compound to be mixed and the refractive index of the compound.

As for the glass powder having X-ray imaging property, if the maximum particle diameter exceeds 5µm, surface smoothness of a dental restoration material composite decreases when the organic and inorganic composite filler is used for the dental restoration material composite. If the average particle diameter is less than 0.05µm, 50% or more by weight of the glass powder cannot be blended in the organic and inorganic composite filler. If the average particle diameter exceeds 2µm, the transparency of the dental restoration material decreases because of being influenced by the refractive index.

The glass powder having a maximum particle diameter of 5µm or less, an average particle diameter from 0.05 to 2µm, and X-ray imaging property is blended in the organic and inorganic composite filler by 50 to 80% by weight. If the blending amount is less than 50% or more than 80% by weight, the mechanical strength of the dental restoration material cannot be improved.

The organic and inorganic composite filler according to the present invention further includes 10 to 40% by weight of a metal compound having an average particle diameter from 0.005 to 0.3µm and X-ray imaging property. As for the metal compound having an average particle diameter from 0.005 to 0.3µm and X-ray impermeability, compounds of alkaline earth metals having atomic numbers larger than 20, such as strontium fluoride, strontium carbonate, barium oxide, and barium carbonate, transition elements having atomic numbers equal to or larger than 39 or its compound such as zirconia, yttrium oxide, yttrium fluoride, and zirconia oxide, and compounds of lanthanoids such as lanthanum fluoride, lanthanum oxide, ytterbium fluoride, and ytterbium oxide, can be used. Further, these can be used by mixing two or more.

An average particle diameter of the metal compound having X-ray imaging property is from 0.005 to 0.3µm. If the average particle diameter is less than 0.005µm, 10% or more by weight of the metal compound cannot be blended in the organic and inorganic composite filler, in addition to a lot of the glass powder having X-ray imaging property. If the average particle diameter exceeds 0.3µm, the transparency of the dental restoration material decreases because of being influenced by refractive index.

If the blending amount of the metal compound having X-ray imaging property in the organic and inorganic composite filler is less than 10% by weight, the effect for improving X-ray imaging property is low. If the blending amount is more than 40% by weight, the amount of the (meth)acrylate compound in the organic and inorganic composite filler decreases so that the organic and inorganic composite filler can not be formed.

It is necessary that the blending amount of the glass powder having a maximum particle diameter of 5µm or less, an average particle diameter from 0.05 to 2µm, and X-ray imaging property and the metal compound having an average particle diameter from 0.005 to 0.3µm and X-ray imaging property in the organic and in organic composite filler is 90% by weight at the maximum. If the blending amount of the glass powder and the metal compound exceeds 90% by weight, the amount of the (meth)acrylate compound in the organic and inorganic composite filler decreases so that the organic and inorganic composite filler can not be formed.

The glass powder having a maximum particle diameter of 5µm or less, an average particle diameter from 0.05 to 2µm, and X-ray imaging property and the metal compound having an average particle diameter from 0.005 to 0.3µm and X-ray imaging property, which are mixed together, are mixed with the (meth) acrylate compound by a mixer, the (meth)acrylate compound is cured, and the obtained product is pulverized so as to produce the organic and inorganic composite filler. As for a curing agent for curing the (meth)acrylate compound, an organic peroxide, an azo compound or the like can be used in a case of thermal-curing. A photopolymerization initiator or the like can be used in a case of optical-curing. In addition, chemical polymerization in ordinary temperature polymerization or the like can be used.

As for the particle size of the organic and inorganic composite filler, an average particle diameter is preferably from 10 to 30µm. If the average particle diameter is less than 10µm, the problem of polymerization shrinkage and the problem of "sticky" are easily caused. If the average particle diameter exceeds 30µm, the surface smoothness of the composite tends to be low. In addition, generally, the blending amount of the organic and inorganic composite filler in the dental restoration material is from 35 to 80% by weight. If the blending amount is less than 35% by weight, the effect for improving the problem of polymerization shrinkage and the problem of "sticky" is low, and if the blending amount exceeds 80% by weight, the operativity of the composite tends to be low.

As for the organic and inorganic composite filler according to the present invention, parts of the glass powder and the metal compound are exposed from the surface after pulverizing. Thus, when the organic and inorganic composite filler is subjected to a surface treatment with a silane coupling agent which is conventionally carried out to a filler, those parts can be strongly bonded to the (meth) acrylate compound at a time of using the filler for the dental restoration material, and thus excellent strength can be given to the dental restoration material.

### [Examples]

The present invention will be described below with examples and comparative examples. In this case, monomers of methacrylate or acrylate and their codes used in examples and comparative examples are shown below.

### UDMA:

### di-2-methacryloxyethyl 2, 2, 4-trimethylhexamethylene dicarbamate

### 3G:

### Triethyleneglycol dimethacrylate

### Bis-GMA:

### 2,2-bis[4-(2-hydroxy-3-methacryloxypropoxy)phenyl] propane

### 1, 3-BG:

### 1,3-butanediol dimethacrylate

### Bis-MPEPP:

### 2,2-bis(4-methacryloxypolyethoxyphenyl)propane

### TMPT:

### Triethyleneglycol trimethacrylate

As for glass powders having a maximum particle diameter of 5µm or less, an average particle diameter from 0.05 to 2µm, and X-ray imaging property, following powders were used.
Glass powder A: Fluoroaminosilicate glass powder (having an average particle diameter of 0.7µm and a maximum particle diameter of 2µm)
Glass powder B: Barium glass powder (having an average particle diameter of 0.4µm and a maximum particle diameter of 1µm)
Glass powder C: Quartz glass powder (having an average particle diameter of 0.7µm and a maximum particle diameter of 2µm)

A composition of the glass powder A will be shown in Table 1. The glass powder A was produced by fully mixing materials shown in Table 1, holding the mixture in a high temperature electric furnace at 1200°C for 5 hours so as to melt the glass; cooling the melted glass, pulverizing the glass for 10 hours by a ball mill, and sieving the pulverized glass with 200-mesh (ASTM). The glass powder B was produced by the same method as the glass powder A except that materials shown in Table 2 were fully mixed and was melted at 1450°C.

**Table 1**

| Fluoroaminosilicate glass powder | |
|---|---|
| Aluminum oxide (g) | 23 |
| Anhydrous silicic acid (g) | 41 |
| Calcium fluoride (g) | 10 |
| Calcium phosphate (g) | 13 |
| Strontium carbonate (g) | 13 |

**Table 2**

| Barium glass powder | |
|---|---|
| Silicon dioxide (g) | 55 |
| Barium oxide (g) | 25 |
| Diboron trioxide (g) | 10 |
| Aluminum oxide (g) | 10 |

As for a metal compound having an average particle diameter from 0.005 to 0.3µm and X-ray imaging property, an ytterbium fluoride powder was used.

### <Examples 1 to 4, and Comparative examples 1 to 4>

An organic and inorganic composite filler of each examples was produced by mixing the glass powder and the metal compound with a (meth)acrylate compound in which 1% by weight of azoisobutyronitrile was added as a curing agent for curing a (meth) acrylate compound, thermally curing the mixture, and pulverizing the cured mixture. Blending compositions and blending amounts will be shown in Table 3 in detail.

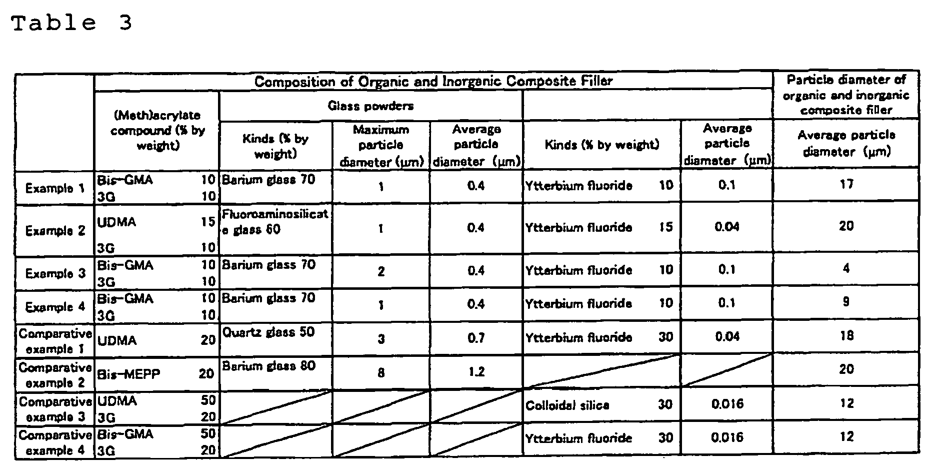

### <Producing of a dental restoration material>

A pasty dental restoration material was produced by mixing a (meth)acrylate compound, each of the organic and inorganic composite fillers of examples and comparative examples, and colloidal silica (the product name: AEROSIL R-972, produced by Nippon Aerosil Corporation) as another fillers and mixing and kneading the mixture in a darkroom. The (meth)acrylate compound was made by dissolving 0.5% by weight of a photosensitizer camphorquinone as a photopolymerization initiator and 1% by weight of dimethylaminoethyl methacrylate as a reducing agent to 100 weight parts of the (meth)acrylate compound. The collidal silica was used for improving dispersibility at a time of the mixing of the organic and inorganic composite fillers and the (meth) acrylate compound. Each dental restoration material obtained was subjected to the following tests.

### (1) Bending strength test

The dental restoration material was pressed to a metal mold having a size of 2mmx2mmx25mm with a glass plate through a cellophane, and light is irradiated to the material from one upper side for 60 seconds by a visible beam irradiator (the product name: G-LIGHT, produced by GC Corporation) so as to subject the whole material to the light. The sample obtained was dipped in water for 24 hours, and subjected to a three-point bending test at a span of 20mm and a crosshead speed of 1 mm/min. by a universal testing machine (AUTOGRAPH, produced by Shimadzu Corporation).

### (2) X-ray imaging property

The dental restoration material was subjected to a test according to ISO4049-2000.

### (3) Ten-point average roughness

The dental restoration material was pressed to a metal mold having an inner diameter of 20mm and a thickness of 2mm with a glass plate through a cellophane, and light is irradiated to the material from one upper side for 60 seconds by a visible beam irradiator (the product name: G-LIGHT, produced by GC Corporation) so as to subject the whole material to the light. The obtained irradiated face of the sample was polished by the Emily Paper No. 600 and thereafter by the Emily Paper No. 1000, and was subjected to a finishing polish using an abrasive (the product name: DIASHINE, produced by GC Corporation). The ten-point average roughness of the finishing-polished surface was measured by a surface roughness testing machine (produced by Kosaka Laboratory Ltd.).

A blending composition and a blending amount of each dental restoration material composite used in examples and comparative examples, and results of each test are shown in Table 4 collectively.

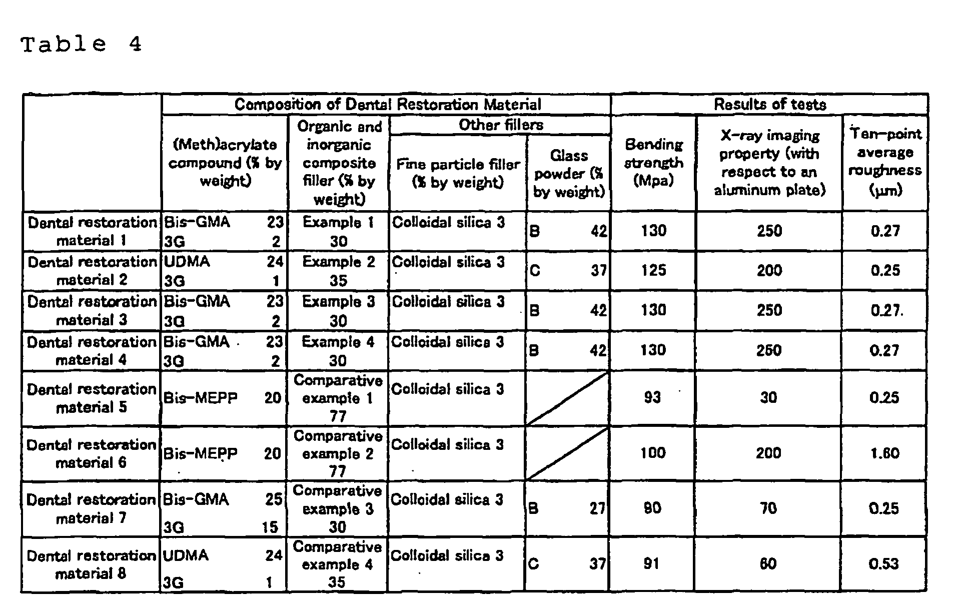

Clearly from Tables 3 and 4, when Examples 1 to 4 of the organic and inorganic composite fillers for the dental restoration material according to the present invention, which was produced by curing and pulverizing the (meth)acrylate compound including 50 to 80% by weight of the glass powder and 10 to 40% by weight of the metal compound having an average particle diameter from 0.005 to 0.3µm and X-ray imaging property at the maximum of 90% by weight in total, were used for the dental restoration material, the dental restoration material had high mechanical strength (bending strength), excellent X-ray imaging property, and low ten-point average roughness. Because of low ten-point average surface roughness, the surface smoothness similar to that of a natural tooth could be acquired.

By contrast, when Comparative example 1 as the conventional organic and inorganic composite filler not having X-ray imaging property, which was produced by thermally curing and pulverizing a mixture of quartz glass, was used for the dental restoration material, the dental restoration material had low mechanical strength (bending strength), and did not have sufficient X-ray imaging property.

When Comparative example 2 of the organic and inorganic composite filler using only the glass powder B having an average particle diameter from 0.05 to 2µm and X-ray imaging property was used for the dental restoration material, the dental restoration material had sufficient X-ray imaging property, but had a high ten-point average roughness. Therefore, the surface smoothness similar to that of a natural tooth could not be acquired.

When Comparative example 3 of the organic and inorganic composite filler, which was produced by mixing a colloidal silica (the product name: AEROSIL R-972, produced by Nippon Aerosil Corporation) not having X-ray imaging property and thermally curing and pulverizing the mixture, was used for the dental restoration material, the dental restoration material had low mechanical strength (bending strength), and did not have sufficient X-ray imaging property.

When Comparative example 4 of the organic and inorganic composite filler, which was produced by mixing only the ytterbium fluoride powder and thermally curing and pulverizing the mixture, was used for the dental restoration material, the dental restoration material had low mechanical strength (bending strength), and did not have sufficient X-ray imaging property.

## Claims

1. An organic and inorganic composite filler for a dental restoration material, produced by curing and pulverizing a (meth)acrylate compound including
(a) 50 to 80% by weight of a glass powder having a maximum particle diameter of 5 µm or less, an average particle diameter from 0.05 to 2 µm, and X-ray imaging property, and including
(b) 10 to 40% by weight of a metal compound having an average particle diameter from 0.005 to 0.3 µm and X-ray imaging property, where the (meth)acrylate compound includes 90% by weight of a combination of the glass powder (a) and the metal compound (b) at the maximum.

2. The organic and inorganic composite filler as claimed in claim 1, wherein said organic and inorganic composite filler has an average particle diameter from 10 to 30 µm.

## Patentansprüche

1. Organischer und anorganischer Compositfüllstoff für ein Zahnrestaurationsmaterial, hergestellt durch Aushärten und Pulverisieren einer (Meth)acrylatverbindung, enthaltend
(a) 50 bis 80 Gew.-% eines Glaspulvers mit einem maximalen Teilchendurchmesser von 5 µm oder weniger, einem mittleren Teilchendurchmesser von 0,05 bis 2 µm und einer Röntgenstrahlen-Abbildungseigenschaft, und enthaltend
(b) 10 bis 40 Gew.-% einer Metallverbindung mit einem mittleren Teilchendurchmesser von 0,005 bis 0,3 µm und einer Röntgenstrahlen-Abbildungseigenschaft, wobei die (Meth)acrylatverbindung 90 Gew.-% einer Kombination aus dem Glaspulver (a) und der Metallverbindung (b) als Maximum enthält.

2. Organischer und anorganischer Compositfüllstoff nach Anspruch 1, wobei der organische und anorganische Compositfüllstoff einen mittleren Teilchendurchmesser von 10 bis 30 µm aufweist.

## Revendications

1. Une charge composite organique et inorganique pour un matériau de restauration dentaire, produite en durcissant et en pulvérisant un composé de (méth)acrylate comprenant
(a) de 50 à 80% en poids d'une poudre de verre ayant un diamètre de particule maximum de 5 µm ou moins, un diamètre moyen de particule de 0,05 à 2 µm, et une propriété d'imagerie aux rayons X, et comprenant
(b) de 10 à 40% en poids d'un composé de métal ayant un diamètre moyen de particule de 0,005 à 0,3 µm et une propriété d'imagerie aux rayons X, dans lequel le composé de (méth)acrylate comprend 90% en poids d'une combinaison de la poudre de verre (a) et du composé de métal (b) au maximum.

2. La charge composite organique et inorganique telle que revendiquée à la revendication 1, dans laquelle ladite charge composite organique et inorganique a un diamètre moyen de particule de 10 à 30 µm.
